Europäisches Patentamt

European Patent Office

Office européen des brevets

(11) Publication number: **0 254 447 B1**

## EUROPEAN PATENT SPECIFICATION

(45) Date of publication of patent specification: **17.03.93**    (51) Int. Cl.⁵: **A61K 7/00**, B01J 13/02

(21) Application number: **87305989.3**

(22) Date of filing: **07.07.87**

(54) **Process for producing polymer-thickened oil macrocapsules.**

(30) Priority: **18.07.86 US 887798**
**18.07.86 US 887799**

(43) Date of publication of application:
**27.01.88 Bulletin 88/04**

(45) Publication of the grant of the patent:
**17.03.93 Bulletin 93/11**

(84) Designated Contracting States:
**DE FR GB IT**

(56) References cited:
**DE-A- 1 619 802**
**DE-A- 1 800 727**
**DE-A- 2 434 406**
**GB-A- 1 304 375**
**US-A- 3 929 988**

(73) Proprietor: **MINNESOTA MINING AND MANU-FACTURING COMPANY**
**3M Center, P.O. Box 33427**
**St. Paul, Minnesota 55133-3427(US)**

(72) Inventor: **Zeller, Lowell C.**
**Minnesota Mining & Manufacturing Co.**
**P.O.Box 33427, St. Paul, MN 55133(US)**
Inventor: **Chang, Robert W.H.**
**Minnesota Mining & Manufacturing Co.**
**P.O.Box 33427, St. Paul, MN 55133(US)**
Inventor: **Norbury Robert J.**
**Minnesota Mining & Manufacturing Co.**
**P.O.Box 33427, St. Paul, MN 55133(US)**

(74) Representative: **Baillie, Iain Cameron et al**
**c/o Ladas & Parry, Altheimer Eck 2**
**W-8000 München 2 (DE)**

Rank Xerox (UK) Business Services
(3.10/3.5x/3.0.1)

**Description**

BACKGROUND OF THE INVENTION

1. Field of the Invention

The present invention relates to a process for producing microcapsules. The microcapsules provide functional additives and can provide mildly abrasive property to the composition upon rupture or rubbing.

2. Background of the Art

It is fairly common to find encapsulated liquid materials in the marketplace. Technology has been available for many years to effectively provide microcapsules with liquid oleophilic ingredients. Representative processes are shown in U.S. Patents 3,016,308 and 3,516,941. These patents disclose in situ polymerization reactions in which a hydrophobic oil phase is dispersed in an aqueous phase containing resin precursors, particularly aminoplast resin precursors (to form urea/aldehyde resins and the like). High shear agitation is used to keep the capsule size small. Addition of an acid catalyst initiates the polycondensation of the aminoplast precursors, resulting in the deposition of the aminoplast resin about the dispersed droplets of the oil phase. This produces the microcapsules.

Other polycondensation encapsulation techniques are shown in U.S. Patents 3,429,827 and 4,000,087. These particular techniques are more limited in the classes of hydrophobic inner phases acceptable in the microcapsules because of reaction with the oil soluble monomer or poor solubility of the monomer in the desired hydrophobic phase.

U.S. Patent 3,930,101 teaches that, to be retained in the hydrophobic phase during high shear dispersion of a fluid particulate dispersion, it is necessary that the particulate be preferentially wetted by the hyrophobic phase. It is suggested to use suitable surfactants which adsorb to the particulate surface as a way to achieve the desired preferential wetting. It has, however, been recognized that, in the in situ polymerization of aminoplast resins method for encapsulation, the presence of surfactants interferes with the deposition of the aminoplast resin at the hydrophobic phase/water phase interface, giving poorly formed or leaky capsules. Similarly, oil soluble suspending agents could alter the wetting of many particulates. Since many of these materials contain carboxylate groups, exposure to highly acidic medias often converts them to carboxylic acid groups altering their adsorbability to the particulates.

U.S. Patent 4,450,221 teaches magnetic toners comprising lyophilic magnetic particles and a resin surrounded by a resin wall to form microcapsules. Colorants such as pigments or dyes may be included in the wall forming resin or the toner. The magnetic particles are rendered lyophilic by treatment with a titanate or silane coupling agent. The coupling agent is said to uniformly disperse the particles in the binder resin and firmly bond the magnetic particle to the resin.

BRIEF DESCRIPTION OF THE INVENTION

Liquid materials such as cosmetic emollient oils generally can be encapsulated by conventional procedures such as shown in U.S. Patent 3,516,941. However, even with careful control of the shear forces in the reaction vessel, the capsules tend to be too small for many commercial applications, particularly in cosmetic applications. The capsules are too difficult to rupture and the broken capsule particles are too small to provide any mildly abrasive benefits and the amount of released functional material is often insufficient to produce the desired benefit.

It has been found that the addition of soluble polymeric materials to the oils enables them to form larger capsules without destroying the properties of the oils. In fact, the polymer also tends to aid the oil in adhering to the surface of skin and penetrating at a more controlled rate over a longer period of time.

It has been found that larger capsules containing oils such as cosmetic ingredients can be dispersed in carrying media and provide additional activity including mild cleaning abrasion.

DETAILED DESCRIPTION OF THE INVENTION

Encapsulated liquids such as oil compositions are added to carrying media such as cosmetic compositions to provide additional effects including mild cosmetic abrasiveness to the overall composition. The encapsulated materials in the form of polymeric microcapsules having average microcapsule diameters between 50 and 2500 $\mu$m (microns) are dispersed within carrying media such as cosmetic compositions such as skin creams, liquid soaps, cleansing gels, cleansing creams, cleansing lotions, lotions, cold creams and the like to provide a beneficial abrasive activity and other cosmetic activity to the composition. Heretofore it has been extremely difficult to provide such large size microcapsules with any production regularity.

These microcapsules are added in weight percentages of 0.5 to 25% by weight of the total cosmetic composition. Preferably the microcapsules are present as from 1 to 15% by weight of the total cosmetic composition.

General types of ingredients in the cosmetic compositions carrying the microcapsules are surfactants, oils, pigments, stabilizers, aromatic oils, water, carboxylic acids, olefin glycols, gelatin, moisturizers, waxes, fragrances, lanolin, and the like.

In compositions used to care for the skin, various oils are used to make lotions, mousses and ointments for both cosmetic and therapeutic applications. The ability to provide additional compositions in microcapsule form enables easy corrections or improvements of compositions, the ability to readily combine functions (e.g., skin softening, insect repellancy and sun screening), and provide a cleansing function to the overall composition by the addition of the large microcapsules.

In accordance with the present invention, microcapsules are prepared by in situ aminoplast polymerization. The techniques disclosed, generally referred to as an in situ polymerization reaction, yield, an aminoplast resin capsule wall material. In the process, a cosmetic emollient oil phase with a viscosity increasing additive dissolved therein is dispersed in an aqueous phase containing the aminoplast resin precursors preferably by applying shear agitation. Addition of an acid catalyst initiates the polycondensation of the aminoplast precursors, resulting in the deposition of the aminoplast resin about the dispersed droplets of the oil phase, producing the microcapsules.

Typical cosmetic emollient oils are organic liquids with viscosities between $2 \times 10^{-3}$ and $150 \times 10^{-3}$ Pas (2 and 150 cP) at 20°C, preferably between $2 \times 10^{-3}$ and $100 \times 10^{-3}$ Pas (2 and 100 cP). The oils preferably have molecular weights in excess of 100, more preferably in excess of 125 and most preferably between 125 and 500. Examples of commercial oils used as cosmetic emollient oils include mineral oil, castor oil, vegetable oil, corn oil, peanut oil, jojoba oil, 2-ethylhexyl oxystearate (and other alkyl oxystearates), acetulated lanolin alcohol, alkyl palmitates such as isopropyl palmitate, 2-ethylhexyl palmitate, glyceral triacetates, disopropyl adipate, dioctyl adipate (and other alkyl adipates), isopropyl myristate, $C_{12}$ to $C_{15}$ alcohol benzoates, and the like.

The viscosity increasing additive is preferably a polymeric additive which must be dispersible or soluble in the oil so as to increase its viscosity. These materials are preferably polymers although waxy substances may be used although with less desirable results. The polymers should be oleophilic to be wetted or soluble in the oil. Examples of preferred polymers include polyolefins, polystyrene, polybutadiene, graft or block polymers of these materials such as a polystyrene-polybutadiene-polystyrene block copolymer, polyacrylates, natural rubber (not heavily vulcanized), polyisoprene, polyisobutylene, cellulose acetate esters such as cellulose acetate butyrate and cellulose acetate proprionate,

The process of the present invention utilizes the addition of viscosity increasing materials selected from the group consisting of particulates (e.g., clays and polymeric particles), waxes, and polymeric additives to cosmetic emollient oils to increase their viscosity and then uses the higher viscosity oil mixtures or solutions in a in-situ amino-polymerization process to produce particles of a larger size than would ordinarily be formed in encapsulation of the cosmetic emollient oil without additives under identical encapsulation reaction conditions. Polymeric additives are especially preferred because they are more consistent and repeatable in their performance and because they hold the oil better on the skin. These oils with increased viscosity are particularly beneficial in encapsulation processes where shear forces are used to maintain a dispersed phase of oil in the reaction vessel. The weight percentage of polymer to oil in the media is generally between 1/2 and 35%, preferably between 5 and 30% by weight of polymer to weight of oil.

The aminoplast shell material of the capsule may be any of the various materials known to be useful in forming such capsules such as urea-aldehydes, and any of the other many well-known aminoplast capsule making materials. The microcapsules have an average diameter of between 50 and 2500 $\mu$m (microns), and more preferably between 100 and 1800 $\mu$m (microns) and most preferably between 200 and 1500 $\mu$m (microns). Preferably they having a loading of (emollient and polymer)/(shell) at least 2:1 and preferably between 3:1 and 10:1. It is particularly advantageous to use shell material which when broken to release their load can act as a mild abrasive. Brittle polymeric materials are therefore especially preferred, and the urea aldehydes are most preferred in that class.

Additional additives such as perfumes, pigments, vitamins, sunscreens, insect repellants and even medication may be added to the oil/polymer mixture or blended with the capsules. These additives, particularly when blended with the capsules after they have been made, may be dispersed in a cream, oil,

3

powder, pancake or other media as a carrier for the capsules. In such media, the capsules would usually constitute from 2 to 50% by weight of the total cosmetic composition, preferably 3 to 40% by weight of the composition, most preferably between 4 and 20% by weight.

Example 1

To 900 grams of a mixture of $C_{12}$-$C_{15}$ alcohol benzoates was added 100 grams of a styrene-butadiene-styrene block copolymer (Kraton[R] 1107). The mixture was heated for four hours at 120°C until the copolymer had dissolved. The thickened oil was encapsulated in a urea-formaldehyde capsule according to the teachings of U.S. Patent No. 3,516,941 with the shear rate controlled to generate capsules having an average diameter between 300 and 400 $\mu$m (microns). These capsules could be rubbed onto the skin, either directly by hand or with a brush applicator and ruptured. The oils would spread evenly on the skin and the broken capsule shells provide a useful, mildly abrasive action on the skin.

Example 2

Seven hundred twenty (720) grams of a mixture of $C_{12}$-$C_{15}$ alcohol benzoates were mixed with 80 grams of the block copolymer of Example 1 and heated to 120°C with stirring until completely dissolved. The solution was cooled to 60°C and 200 grams of a commercially available bactericide (Irgason[R]-300) was added to 790 grams of the solution. This mixture was cooled to 40°C and 10 grams of fragrance was added with stirring. This solution was then encapsulated according to the procedures of Example 1. The capsules were useful as a directly applied underarm deodorant composition. The capsules could also be blended with a wax or cream to form a composition then could be applied to the underarms. The natural movement of the arms is sufficient to rupture the capsules over a period of time.

Example 3

Twenty-five (25) grams of the copolymer of Example 1 were dissolved in 975 grams of 2-ethylhexyl oxystearate. The mixture was heated to 100°C with stirring and dissolved in the manner described below.

The details of the encapsulation process are as follows:

To a one-liter baffled reactor were charged 379 gm urea-formaldehyde precondensate and 181 gm water. Vigorous mixing was applied and 80.1 gm sodium chloride and 0.53 gm sodium carboxymethyl cellulose were added. To the reactor was then added 250.8 gm of the fill material of Example 1 and precise temperature and mixing speed were applied. Sulfuric acid catalyst was added to achieve a pH of 2.5. This condition was held for two hours followed by an increase in temperature to 60°C (140°F) for 2 hours. The reaction was cooled to room temperature and neutralized to a pH of 8.0. The resulting capsules were filtered, washed, and dried. The excellent quality capsules were determined to have a median size of 354 $\mu$m (microns).

To a one liter baffled reactor were charged 303.2 gm urea-formaldehyde precondensate and 221 gm water. Vigorous mixing was applied, followed by the addition to the reactor of 37.8 gm sodium sulfate and 0.5 gm sodium carboxymethyl cellulose. After achieving solution 297 gm of the fill material, as of Example 2, was added. Precise mixing speed and temperature control were applied followed by the addition of sulfuric acid to pH 2.3. Conditions were held for three hours followed by temperature increase to (140°F) 60°C for two hours. The excellent quality capsules having a median size of 61 $\mu$m (microns) were filtered, washed, and dried to a slightly clumped product.

To a 19 liter baffled reactor were added 7525 gm urea-formaldehyde precondensate and 4000 gm water. Vigorous mixing was applied followed by addition of 1650 gm sodium chloride and 11.0 gm sodium-carboxymethyl cellulose. After obtaining solution 4465 gm of the fill Of Example 3 was added. Precise temperature and turbine speed controls were established, followed by addition of dilute hydrochloric acid to a pH of 2.31. This condition was held for two hours followed by a temperature increase to 60°C (140°F) for 1.75 hours. The resulting capsules having a median size of 330 $\mu$m (microns) were of excellent quality.

Examples 4 and 5

Example 4: To 215.9 gm of isopropyl palmitate having a measure viscosity of $8 \times 10^{-3}$ Pas (8 centipoise) was added with stirring 71.9 gm of Amoco Indopol H-100 polybutene. The resulting mixture had a measured viscosity of $24 \times 10^{-3}$ Pas (24 centipoise) and was encapsulated.

Example 5a: To 180 ml refined jojoba bean oil having a viscosity of $33 \times 10^{-3}$ Pas (33 centipoise) was added 70 ml Amoco Indopol H-100 polybutene. The resulting mixture had a measured viscosity of $106 \times 10^{-3}$ Pas (106 centipoise) and was encapsulated.

Example 5b: 226.1 gm Carnation Mineral Oil and 2.3 gm Kraton 1107 were charged to a wide-mouth jar and were alternately heated on a steam bath and shaken until solution was achieved. The viscosity of the mineral oil increased from its initial value of $19 \times 10^{-3}$ Pas (19 centipoise) to $460 \times 10^{-3}$ Pas (460 centipoise) with the Kraton. This was encapsulated.

The encapsulation process for these oils was the same as described above.

## Examples 6 - 10

These examples show the effectiveness of viscosity increasing additives in generating larger capsule shells under otherwise identical reaction conditions.

## Example 6

To a one liter baffled reactor were charged 379 gm urea-formaldehyde precondensate and 181 gm water. Vigorous mixing was applied and 80.1 gm sodium chloride and 0.53 gm sodium carboxymethyl cellulose were added. To the reactor was then added 250.8 gm of the fill material described in Example 1 and precise temperature and mixing speed were applied. Sulfuric acid catalyst was added to achieve a pH of 2.5. This condition was held for two hours followed by an increase in temperature to 60°C (140°F) for two hours. The reaction was cooled to room temperature and neutralized to a pH of 8.0. The resulting capsules were filtered, washed, and dried. The excellent quality capsules were determined to have a mdian size of 354 $\mu$m (microns). A similarly-run encapsulation reaction using the unviscofied fill material yielded a median capsule size of 155 $\mu$m (microns).

## Example 7

To a one liter baffled reactor were charged 303.2 gm urea-formaldehyde precondensate and 221 gm water. Vigorous mixing was applied followed by addition to the reactor of 37.8 gm sodium sulfate and 0.5 gm sodium carboxymethyl cellulose. After achieving solution, 297 gm of the fill material as described in Example 2 above was added. Precise mixing speed and temperature control were applied followed by addition of sulfuric acid to pH 2.3. Conditions were held for three hours followed by temperature increase to 60°C (140°F) for two hours. The excellent quality capsules having a median size of 61 $\mu$m (microns) were filtered, washed, and dried to a slightly clumped product. Capsules similarly prepared using unviscofied fill had a median size of 32 $\mu$m (microns).

## Example 8

To a 19 liter baffled reactor were added 7525 gm urea-formaldehyde precondensate and 4000 gm water. Vigorous mixing was applied followed by addition of 1650 gm sodium chloride and 11.0 gm sodium carboxymethyl cellulose. After obtaining solution 4465 gm of the fill as described in Example 3 were added. Precise temperature and turbine speed controls were established followed by addition of dilute hydrochloric acid to a pH of 2.31. This condition was held for two hours followed by a temperature increase to 60°C (140°F) for 1.75 hours. The resulting capsules having a median size of 330 $\mu$m (microns) were of excellent quality. Capsules of unviscofied fill from a similar encapsulation reaction had a median size of 145 $\mu$m (microns).

## Example 9

To a one liter baffled reactor were charged 376 gm urea-formaldehyde precondensate and 200 gm water. Vigorous mixing was applied followed by addition to the reactor of 82.5 gm sodium chloride and 0.55 gm sodium carboxymethyl cellulose. After obtaining solution, 216 gm of the mixture as described in Example 4 above was added. Precise mixing speed and temperature controls were applied followed by addition of hydrochloric acid to pH of 2.3. This condition was held for two hours followed by an increase in temperature to 60°C (140°F) for 1.9 hours. The resulting capsules were of excellent quality and had a median size of 186 $\mu$m (microns). Unviscofied fill when encapsulated via a similar method yielded capsules having a median size of 146 $\mu$m (microns).

## Example 10

An encapsulation was performed using the procedure as summarized in Example 9 but substituting 250 ml of fill as described in Example 5a. The resulting capsules were of excellent quality having a median size of 249 $\mu$m (microns). The unviscofied fill yielded capsules of 170 $\mu$m (microns). median size.

Through the procedure in Example 9 using 250 ml of the mineral oil/Kraton as described in Example 5b as the fill material. The resulting capsules were of varying quality and had an average size of 315 $\mu$m (microns).

## Example 11

Cleansing gel with microcapsules.

|  |  | Parts by weight |
|---|---|---|
| A: | Tauranol WS Conc. (sodium methyl cocoyl taurate)<br>Emcol NA-30 (sodium cocamidopropyl betaine)<br>Triethanolamine (TEA)<br>Water | 20.0<br>20.0<br>3.2<br>16.0 |
| B: | Surfine AZI-A (Nonoxynol-10-carboxylic acid)<br>$NH_4Cl$<br>Water | 20.0<br>0.6<br>20.2 |
| C: | Microcapsules of Example 1 | 5.3 |

Part A was mixed and heated with agitation to 70°C. Part B was separately mixed with agitation to 70°C until homogenous and fluid. B was added to A with continued stirring until clear. The mixture was kept warm at 55-60°C until bubbles rose. C was added with minimum agitation to give 5% by weight of microcapsules in the above cleanser. The mixture was allowed to cool to room temperature and then capsules were suspended in the cleanser gel.

## Example 12

Commercial cleanser with microcapsules.

Noxzema skin cream (Noxell Corp) - Active ingredients listed on label: camphor, phenol (less than 1/2%), olive oil, eucalyptus oil, and menthol. Also contains: water, stearic acid, linseed oil, soybean oil, fragrance, propylene glycol, gelatin, ammonium hydroxide, calcium hydroxide.

| Noxzema skin cream | 38 g |
|---|---|
| Microcapsules of Example 1 | 2 g |

The materials were mixed well with a spatula and the microcapsules remained suspended in the cream.

## Example 13

Pond's Lemon cold cream (Cheeseborough Ponds Inc.)
Contents listed on label: mineral oil, water, beeswax, ceresin, sodium borate, fragrance, carbomer-934.

| Pond's Lemon cold cream | 38 g |
|---|---|
| Microcapsules of Example 1 | 2 g |

Example 14

|  | Parts by weight |
|---|---|
| Finsolv TN | 4.0 |
| Lipocol L-23 (Laureth-23) | 1.0 |
| Galactosol 416 | 1.2 |
| Water | 93.8 |

Water was heated to 70°C and then slowly added Galactosol 416 with high sheer mixing until it formed a uniform thick gel. Heated Finsolv TN and Lipocol L-23 and added them to the hot gel with good sheer mixing. This was cooled to 50°C and then added microcapsules of Example 1 in an amount equal to 5% of the warm gel.

Example 15

Facial Scrub Cream - The following composition was prepared by mixing of the ingredients listed.

## Example 15

Facial Scrub Cream — The following composition was prepared by mixing of the ingredients listed.

|  | Parts by weight |
|---|---|
| Deionized water | 47.91 |
| Sodium lauroyl sarcosinate (30%) | 9.5 |
| Sodium cocoyl isethionate (80%) | 5.7 |
| Sodium laureth sulfate (26%) | 5.5 |
| Mixture of fatty alcohol ether sulfates (30%) | 4.8 |
| Cocamidopropyl hydroxysultaine (43%) | 4.8 |
| Lauroamphocarboxyglycinate (30%) | 2.9 |
| Glyceryl stearate (and) PEG-100 (polyethyleneglycol) stearate | 4.8 |
| Lauric/linoleic diethanolamide | 1.4 |
| Laureth-4 (4 unit polyethylene glycol laurate) | 1.2 |
| Cetearyl alcohol (and) polysorbate 60 | 0.9 |
| PPG-15 stearyl ether | 1.2 |
| Shea butter | 0.9 |
| Magnesium aluminum silicate | 1.7 |
| Latex opacifier | 0.9 |
| Preservative (methyl and propal paraben) | 0.25 |
| Fragrance | 0.10 |
| Lactic acid | 0.54 |
| Microcapsules of Example 3 | 5.0 |

EP 0 254 447 B1

Example 16

Facial scrub gel - The following cosmetic composition was prepared by mixing of the ingredients listed.

|  | Parts by weight |
|---|---|
| a) Deionized water | 58.71 |
| b) Lauroamphocarboxyglycinate (30%) | 11.4 |
| c) Ammonium lauryl sulfosuccinate (40%) | 11.0 |
| d) Cocamidopropyl hydroxysultaine (43%) | 9.3 |
| e) Cocamide DEA | 1.4 |
| f) Carbomer 1342 (water-swellable acrylic polymer) | 0.83 |
| g) Ammonium hydroxide conc. | 0.67 |
| h) 0.1% FD&C Blue No. 1 | 0.39 |
| i) Preservative (methyl and propyl paraben) | 0.25 |
| j) Fragrance | 0.05 |
| k) Microcapsules of Example 3 | 6.0 |

The procedure for making such gels can be exemplified by the following steps used in making this particular cosmetic composition:

The acrylic polymer was slowly added with brisk mixing for five minutes to the water which was preheated to 50°C. Mixing was continued for five minutes at 82°C. A preheated (82°C) mixture of components b, c, d and e were then added with mixing. The ammonium hydroxide was then used to adjust the pH to 5.8-6.2. Component h was added and the mixture gradually cooled. At 50°C i and j were added and mixed for a few minutes. Then k was added with slow mixing for another two minutes and the gel was formed upon cooling.

Examples 17-19

a) 352 gm of N,N-diethyl toluamide (DEET) was mixed with heating with 48 gm Kraton[R] 1107 until dissolved.

The viscosity of the resulting solution measured $91 \times 10^{-3}$ Pas (91 centipoise), unviscofied DEET measured $17.5 \times 10^{-3}$ Pas (17.5 centipoise).

b) 225 gm of a commercially purchased oil base wood stain having a measured viscosity of $4 \times 10^{-3}$ Pas (4 centipoise) was mixed and heated with 25 gm Kraton[R] 1107 until solution was achieved. This mixture had a viscosity of $74 \times 10^{-3}$ Pas (74 centipoise).

c) 368 gm of Escalol 507 (2-ethyl hexyl para-dimethylaminobenzoate) was stirred with 32 gm Kraton[R] 1107 while being heated on a steam bath until a solution was achieved. The resulting solution has a measured viscosity of 1.24 Pas (1240 centipoise) compared with a viscosity of $60 \times 10^{-3}$ Pas (60 centipoise) for the unviscofied Escalol.

Example 17

As in the procedure used in Example 9, using the fill material a) shown above, capsules with an average diameter of 101 $\mu$m (microns) were produced. The unviscofied DEET provided capsules of 81 $\mu$m (microns) under otherwise identical conditions.

Example 18

Using the procedures of Example 9 with the fill material b) described above, capsules with an average diameter of 265 $\mu$m (microns) were produced. The unviscofied wood stain provided capsules with average diameter of 127 $\mu$m (microns) under otherwise identical conditions.

Example 19

Using the procedure of Example 9 with 250 ml of the Escalol/Kraton as described in c) above as the fill material, microcapsules were formed. The resulting capsules were of varying quality and had a median volume size of 326 $\mu$m (microns); unviscofied fill encapsulated in similar fashion yielded capsules of 165

8

μm (micron) size.

## Claims

1. A process for producing microcapsules having an average diameter between 50 and 2500 μm and comprising emollient oils encapsulated in an aminoplast resin which process comprises mixing an emollient oil having a viscosity of between $2 \times 10^{-3}$ and $150 \times 10^{-3}$ Pas (2 and 150 cP) at 20°C and a molecular weight in excess of 100 with an additive that increases its viscosity and which is dispersible or soluble in said emollient oil, then encapsulating said emollient oil with said additive by means of an in situ aminoplast polymerization to produce microcapsules having an average diameter of between 50 and 2500μm (microns).

2. The process of claim 1 wherein said microcapsules have an average diameter of between 200 and 1500 μm (microns).

3. The process of any preceding claim wherein said emollient oil plus said viscosity increasing additive has a viscosity between 0.3 and 1.5 Pas (300 and 1500 cP) at 20°C.

4. The process of any preceding claim wherein said viscosity increasing additive is a polymeric thickening agent.

5. The process of claim 4 wherein said polymeric thickening agent is selected from polyolefins, polybutadiene, polystyrene, polyacrylics, gelatin, natural rubber, polyisoprene, cellulose acetate esters and copolymers thereof.

6. The process of any preceding claim wherein said emollient oil is selected from at least one of mineral oil, castor oil, jojoba oil, vegetable oil, 2-ethylhexyl oxystearate, $C_{12}$-$C_{15}$ alcohol benzoates, isopropyl palmitate and isopropyl myristate.

## Patentansprüche

1. Verfahren zum Erzeugen von Mikrokapseln, die einen durchschnittlichen Durchmesser zwischen 50 und 2500 Mikrometern haben und in denen weichmachende Öle in einem Aminoplastharz eingekapselt sind, wobei in dem Verfahren ein weichmachendes Öl mit einer Viskosität zwischen $2.10^{-3}$ und $150.10^{-3}$ Pa.s und einem Molekulargewicht über 100 mit einem viskositätserhöhenden Zusatzstoff gemischt wird, der in dem weichmachenden Öl dispergierbar oder löslich ist, und danach zur Bildung von Mikrokapseln mit einem durchschnittlichen Durchmesser zwischen 50 und 2500 Mikrometern das den Zusatzstoff enthaltende weichmachende Öl durch in situ durchgeführte Aminoplastpolymerisation eingekapselt wird.

2. Verfahren nach Anspruch 1, dadurch gekennzeichnet, daß die Mikrokapseln einen durchscnittlichen Durchmesser zwischen 200 und 3500 Mikrometern haben.

3. Verfahren nach einem der vorhergehenden Ansprüche, dadurch gekennzeichnet, daß das den viskositätserhöhenden Zusatzstoff enthaltende weichmachende Öl eine Viskosität zwischen 0,3 und 1,5 Pa.s hat.

4. Verfahren nach einem der vorhergehenden Ansprüche, dadurch gekennzeichnet, daß der viskositätserhöhende Zusatzstoff ein polymeres Verdickungsmittel ist.

5. Verfahren nach Anspruch 4, dadurch gekennzeichnet, daß das polymere Verdickungsmittel aus den Polyolefinen, dem Polybutadien, Polystyrol, den Polyacrylharzen, der Gelatine, dem Naturkautschuk, dem Polyisopren, den Calluloseacetatestern und deren Copolymeren ausgewählt ist.

6. Verfahren nach einem der vorhergehenden Ansprüche, dadurch gekennzeichnet, daß das weichmachende Öl aus wenigstens einer der nachstehenden Stoffe ausgewählt ist: Mineralöl, Ricinusöl, Jojobaöl, Pflanzenöle, 2-Ethylhexyloxystearat, $C_{12}$-$C_{15}$-Alkoholbenzoate, Isopropylpalmitat und Isopropylmyristat.

**Revendications**

1. Procédé pour la préparation des microcapsules ayant un diamètre moyen compris entre 50 et 2500 $\mu$m et comprenant des huiles émollientes encapsulées dans une résine aminoplaste, ledit procédé comprenant le mélange d'une huile émolliente ayant une viscosité comprise entre 2 x 10$^{-3}$ Pas et 150 x 10$^{-3}$ Pas (2 et 150 cP) à 20°C et un poids moléculaire supérieur à 100 avec un additif augmentant sa viscosité et qui peut être dispersé ou dissous dans ladite huile émolliente, l'encapsulation de ladite huile émolliente avec ledit additif par polymérisation in situ d'un aminoplaste afin de produire des microcapsules ayant un diamètre moyen compris entre 50 et 2500 $\mu$m (microns).

2. Procédé selon la revendication 1, dans lequel lesdites microcapsules ont un diamètre moyen compris entre 200 et 1500 $\mu$m (microns).

3. Procédé selon l'une quelconque des revendications précédentes, dans lequel ladite huile émolliente ayant reçu ledit additif augmentant la viscosité, présente une viscosité comprise entre 0,3 et 1,5 Pas (300 et 1500 cP) à 20°C.

4. Procédé selon l'une quelconque des revendications précédentes, dans lequel ledit additif augmentant la viscosité est un agent épaississant polymère.

5. Procédé selon la revendication 4, dans lequel ledit épaississant polymère est choisi parmi les polyoléfines, le polybutadiène, le polystyrène, les polyacryliques, la gélatine, le caoutchouc naturel, le polyisoprène, les esters d'acétate de cellulose et les copolymères de ceux-ci.

6. Procédé selon l'une quelconque des revendications précédentes, dans lequel ladite huile émolliente est choisie parmi au moins l'un des produits suivants: huile minérale, huile de ricin, huile de jojoba, huile végétale, oxystéarate de 2-éthylhéxyle, benzoate d'alcools en $C_{12}$-$C_{15}$, palmitate d'isopropyle et myristate d'isopropyle.